Europäisches Patentamt

(19) European Patent Office

Office européen des brevets

(11) Numéro de publication : **0 036 095**
**B1**

(12) # FASCICULE DE BREVET EUROPÉEN

(45) Date de publication du fascicule du brevet :
09.01.85

(51) Int. Cl.⁴ : **A 61 B 5/04**, A 61 B 3/10

(21) Numéro de dépôt : **81101211.1**

(22) Date de dépôt : **20.02.81**

(54) **Dispositif d'électrode pour mesurer des potentiels électriques de l'oeil.**

(30) Priorité : **14.03.80 CH 2009/80**

(43) Date de publication de la demande :
**23.09.81 Bulletin 81/38**

(45) Mention de la délivrance du brevet :
**09.01.85 Bulletin 85/02**

(84) Etats contractants désignés :
**DE FR GB SE**

(56) Documents cités :
**DE-A- 431 950**
**DE-A- 865 935**
**DE-A- 2 639 635**
**FR-A- 2 443 233**
**US-A- 4 109 648**
**IEEE TRANSACTIONS ON BIOMEDICAL ENGINEER-
ING, vol. BME 22, no. 5, septembre 1975, New York,
US, A. TROELSTRA et al.: "The Electrical Response
of the Human Eye to Sinusoidal Light Stimulation",
pages 369-378**

(73) Titulaire : **Grounauer, Pierre-Alain
38, rue de l'Ale
CH-1003 Lausanne, Vaud (CH)**

(72) Inventeur : **Grounauer, Pierre-Alain
38, rue de l'Ale
CH-1003 Lausanne, Vaud (CH)**

(74) Mandataire : **Micheli, Michel Pierre et al
MICHELI & CIE 118, Rue du Rhône Case Postale 47
CH-1211 Genève 6 (CH)**

Jouve, 18, rue St-Denis, 75001 Paris, France

## Description

La présente invention se rapporte à un dispositif d'électrodes pour mesurer des potentiels électriques apparaissant sur la cornée de l'œil humain ou animal en réponse à une stimulation lumineuse dudit œil en vue de l'établissement d'un électrorétinogramme selon le préambule de la revendication 1.

Un tel dispositif est connu du brevet USA N° 4.109.648. Dans ce dispositif connu l'électrode est formée par un anneau de carbone qui est recouvert par une couche d'hydrogel déshydraté qui doit être réhydraté avant l'emploi.

On sait que la stimulation lumineuse de l'œil humain ou animal modifie le comportement électrique des cellules photoréceptrices. Après une photostimulation appropriée, il est donc possible de mesurer, dans des conditions normales ou pathologiques, ces caractéristiques électriques, dont l'étude permet, en médecine ophtalmologique, de savoir par exemple si le sujet voit ou non (examens réalisés sur des nouveau-nés ou sur des animaux) ou si la rétine est encore en bon état (bilan pré-opératoire). Pour recueillir ces caractéristiques électriques, il y a lieu de mesurer les potentiels électriques au moyen d'une électrode appliquée directement sur la cornée de l'œil, puis d'amplifier les signaux reçus et de les enregistrer graphiquement en fonction du temps ou de les visualiser sur un écran cathodique.

On connaît déjà des dispositifs avec électrode de mesure à mettre en contact électrique avec le globe oculaire. Toutefois, ces dispositifs actuellement utilisés présentent les désavantages suivants :

— ils sont lourds et encombrants, ce qui constitue un grave inconvénient pour les examens réalisés sur les nouveau-nés et les enfants et un caractère inconfortable pour les examens sur les adultes ;

— l'électrode de mesure n'est pas en contact direct avec la cornée de l'œil, ce qui nécessite la présence entre l'œil et l'électrode d'un liquide de contact conducteur, ce liquide ne devant en aucun cas présenter de bulles d'air ce qui est difficile à obtenir de façon constante ;

— étant donné leur relative complexité, ces dispositifs sont relativement onéreux et sont conçus pour être utilisés plusieurs fois ; or, ils sont difficiles à stériliser rapidement et facilement entre deux mesures, et leur utilisation répétée présente donc des risques non négligeables de contamination.

Le but de cette invention telle qu'elle est revendiquée consiste par conséquent à fournir un dispositif de mesure des potentiels électriques apparaissant sur la cornée de l'œil humain ou animal en réponse à une stimulation lumineuse dudit œil en vue de l'établissement d'un électrorétinogramme, qui obvie aux inconvénients précités et plus particulièrement qui soit simple, léger, peu encombrant, bon marché, facilement stérilisable et prévu en principe pour une utilisation unique.

Il est connu du brevet DE-A 431.950 de prévoir sur la surface convexe d'une lentille cornéenne des ergots pour supporter la paupière supérieure.

Le dessin annexé illustre schématiquement et à titre d'exemple deux formes d'exécution de certaines caractéristiques selon l'invention.

La figure 1 est une vue de dessus d'une première réalisation, et la figure 2 est une vue en coupe selon la ligne I-I de la figure 1.

La figure 3 est une vue d'une seconde réalisation en position de service sur la cornée d'un œil.

La figure 4 est un schéma bloc d'un appareillage pour l'établissement d'un électrorétinogramme.

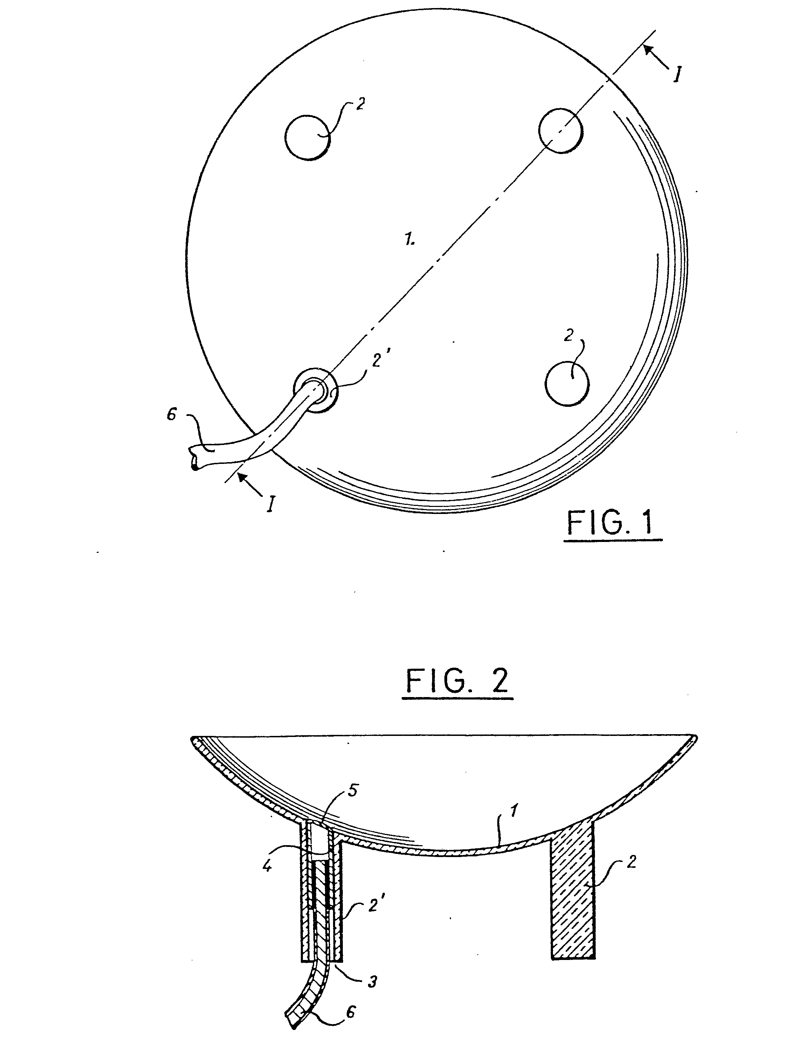

Comme illustré sur les figures 1 et 2, le dispositif comprend une lentille de contact 1 de type conventionnel réalisée en une matière plastique neutre chimiquement et optiquement, transparente et dont le rayon de courbure est approprié pour un bon contact avec la cornée de l'œil.

Sur la surface convexe de la lentille 1 sont disposés quatre pitons ou ergots 2, également réalisés en matière plastique, qui servent de blépharostat, donc de moyens pour maintenir, lorsque la lentille est en position de service sur la cornée, les paupières de l'œil écartées. Un de ces ergots 2' est percé d'un passage axial 3. Ces ergots 2, 2' sont généralement réalisés d'une pièce de fabrication par injection de matière plastique.

Dans le passage 3 de l'ergot 2' est chassée une tige ou un petit tube métallique 4 de telle sorte que l'extrémité fermée 5 de ce tube 4 soit légèrement en saillie par rapport à la surface concave de la lentille 1. Cette portion en saillie 5 du tube 4 est arrondie, de manière à ce qu'en position de service de la lentille sur l'œil, elle ne risque pas de blesser la cornée.

Un fil de contact 6 est connecté, par exemple par soudure, au tube 4, et relie celle-ci à un appareil de mesure des potentiels électriques, qui amplifie et transmet les signaux reçus à un enregistreur graphique ou à un écran cathodique.

Toutefois, l'électrode se présente sous la forme d'un revêtement métallique partiel ou complet de cette surface concave, le fil de contact avec ce revêtement servant d'électrode sortant comme précédemment de la lentille par un passage pratiqué à travers un des ergots. Ce revêtement conducteur peut être obtenu, de façon connue, par exemple par métallisation sous vide ou par réaction chimique en phase gazeuse (CVD).

L'électrode doit être neutre, sans effet photo-électrique et non polarisable, de manière à empêcher tout risque de réaction électrochimique. Elle est donc généralement constituée par un métal noble tel que l'or ou l'argent, ou au moins revêtue par un tel métal.

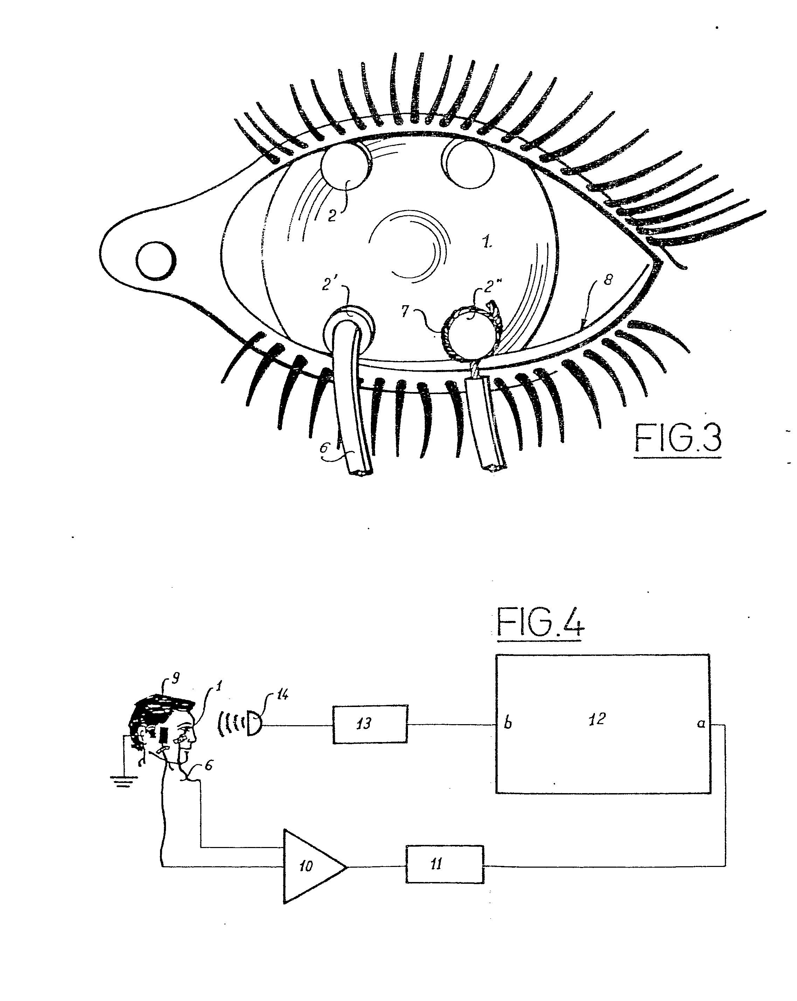

Pour l'obtention d'un électrorétinogramme, il est nécessaire, en plus de l'électrode de mesure, de prévoir d'une part une électrode de référence ou inactive et d'autre part d'une électrode de mise à terre, qui sont fixées pour l'examen par exemple respectivement sur l'oreille ou la tempe et sur le front. Dans la forme d'exécution du dispositif selon l'invention illustrée sur la figure 3, l'électrode de référence ou inactive est constituée par un fil conducteur 7 fixé autour d'un des ergots 2", autre que celui à travers lequel passe le fil de contact 6 de l'électrode de mesure. Dans la position de service illustrée, la lentille 1 étant placée sur le globe de l'œil, l'électrode inactive ou de référence 7 est en contact avec la paupière inférieure 8.

D'une manière générale, tant le fil de contact 6 de l'électrode de mesure que le fil conducteur 7 constituant l'électrode inactive ou de référence sont de préférence blindés afin de diminuer la formation d'effets parasites susceptibles de perturber les signaux captés. De plus, l'extrémité libre de ces fils peut être munie d'une fiche appropriée pour pouvoir être directement introduite dans l'entrée correspondante de l'appareil auquel ils sont reliés.

La figure 4 illustre un schéma bloc simplifié montrant comment est obtenu un électrorétinogramme. La lentille 1 est disposée sur l'œil du sujet 9 à examiner et le fil de contact 6 de l'électrode de mesure est branché sur un amplificateur physiologique 10, de même qu'une électrode inactive ou de référence fixée par exemple sur la tempe du sujet. L'appareillage de mesure comprend également un dispositif de filtrage 11 relié à la sortie de l'amplificateur 10 et destiné à optimaliser le rapport signal/bruit de fond, avant d'atteindre l'ensemble « moyenneur » de signaux 12. Celui-ci comprend d'une part un dispositif d'enregistrement graphique ou de visualisation sur écran (a) des signaux provenant du dispositif de filtrage 11 et d'autre part un dispositif de commande des stimulations lumineuses (b). Les stimulations lumineuses sont fournies par un dispositif stimulateur comprenant une unité de commande et de réglage 13 et une source lumineuse 14 dirigée vers les yeux d'un sujet 9.

Le principe et le fonctionnement de l'appareillage sont décrits plus en détails dans la publication « Introduction to evoked potential instrumentation » de Nicolet Instrument Co (Madison, Wisc., USA/Mars 1, 1979).

La stimulation lumineuse peut être continue ou discontinue. Dans le premier cas, une fréquence lumineuse sinusoïdale peut être superposée, comme décrit par V. V. Toi et P. A. Grounauer dans Rev. Sci. Instrum. 49 (10), 1403-1406, 1978. Dans le second cas, la source lumineuse peut être constituée par un flash électronique pour l'examen de l'ensemble de l'œil ou par une source ponctuelle pour l'examen d'une portion déterminée de l'œil. Un stimulateur du type de celui décrit par V. V. Toi et P. A. Grounauer dans Klin. Mbl. Augenheilk. 176 (4), 530-532, 1980 peut être utilisé.

Par rapport aux dispositifs connus, le dispositif selon l'invention présente l'avantage d'être constitué par une lentille cornéenne, donc d'être léger et facilement supportable par le sujet examiné, même si celui-ci est un enfant. Cette lentille peut présenter une transparence maximale de tout le spectre visible et avoir ainsi un pouvoir optique neutre ne modifiant pas l'acuité visuelle.

De plus, l'électrode de mesure étant directement en contact avec la cornée de l'œil, il n'est pas nécessaire de prévoir un liquide de contact conducteur entre l'électrode et l'œil.

Enfin, ce dispositif étant simple et aisé à fabriquer, il est d'un prix de revient très peu élevé et peut par conséquent être prévu sous une forme non réutilisable, jetable, ce qui évite tout risque de contamination d'un patient à l'autre. Par exemple la lentille cornéenne avec électrode de mesure peut être commercialisée sous emballage scellé et après stérilisation selon une méthode connue par traitement radioactif sous forme emballée. Ainsi, l'ophtalmologue peut réaliser des examens par mesure des potentiels électriques directement sur la cornée de l'œil en utilisant pour chaque sujet examiné une nouvelle lentille qu'il ne sortira qu'au dernier moment de son emballage stérile et qu'il jettera après la mesure.

### Revendications

1. Dispositif à électrode pour la mesure des potentiels électriques apparaissant sur la cornée de l'œil humain ou animal en réponse à une stimulation lumineuse dudit œil en vue de l'établissement d'un électrorétinogramme consistant en une lentille cornéenne (1) munie d'une électrode de mesure sur la surface concave de la lentille, caractérisé par le fait que cette électrode est consituée par un revêtement métallique conducteur non polarisable dont est munie en totalité ou en partie ladite surface concave et qui en position de service est directement en contact avec la cornée de l'œil, et par le fait que la surface convexe de la lentille comporte des ergots (2, 2', 2") pour maintenir, en position de service, les paupières de l'œil écartées.

2. Dispositif selon la revendication 1, caractérisé par le fait qu'un fil de contact (6, 4) passe par une ouverture axiale pratiquée dans un desdits ergots (2'), pour relier l'électrode à un appareil de mesure.

3. Dispositif selon la revendication 1 ou la revendication 2, caractérisé par le fait que la lentille cornéenne et les ergots sont venus d'une pièce de fabrication par injection de matière plastique.

4. Dispositif selon l'une des revendications 1 à 3, caractérisé par le fait qu'il comporte un fil conducteur (7), qui est fixé autour d'un des ergots (2") autre que celui (2') par lequel passe ledit fil de contact (6, 4), ce fil conducteur (7) servant en position de service d'électrode inactive ou de référence.

### Claims

1. Device with electrode for the measurement of electrical potentials appearing on the cornea of the human or animal eye in response to a light stimulation of said eye and so as to establish an electroretinogram consisting in a corneal lens (1) provided with a measurement electrode on the concave surface of the lens, characterized by the fact that said electrode is constituted of a non polarizable conductor metallic coating with which said concave surface is completely or partially provided and which in service position is directly in contact with the cornea of the eye, and by the fact that the convex surface of the lens comprises pins (2, 2', 2") for maintaining in service position the eyelids opened.

2. Device according to claim 1, characterized by the fact that a contact wire (6, 4) is passing through an axial boring in one of said pins (2'), so as to connect the electrode to a measuring apparatus.

3. Device according to claim 1 or claim 2, characterized by the fact that the corneal lens and the pins are made of one piece of manufacture by injection of plastic material.

4. Device according to one of claims 1 to 3 characterized by the fact that it comprises conductor wire (7) which is attached to one of the pins (2") other than (2') that through which said contact wire (6, 4) passes, said conductor wire (7) serving in service position as inactive or reference electrode.

**Ansprüche**

1. Elektrodenvorrichtung zum Messen der elektrischen Augenpotentiale auf der Hornhaut von Menschen- oder Tieraugen, die als Reaktion auf einem zur Erstellung eines Elektroretinogramms erzeugten Lichtreiz des Auges auftreten, mit einer Hornhautlinse (1), die auf ihrer konkaven Oberfläche eine Meßelektrode aufweist, dadurch gekennzeichnet, daß die Elektrode aus einem nicht polarisierbaren leitenden Metallüberzug besteht, der die konkave Fläche ganz oder teilweise bedeckt, und die bei der Anwendung direkt auf der Hornhaut des Auges aufliegt, und daß die konkave Linsenfläche Stifte (2, 2', 2") aufweist, die während der Anwendung die Augenlider geöffnet halten.

2. Vorrichtung nach Anspruch 1, dadurch gekennzeichnet, daß eine Kontaktleitung (6, 4) durch eine in einen der Stifte (2') eingearbeitete axial Öffnung geführt ist, um die Elektrode an einen Meßapparat anzuschliessen.

3. Vorrichtung nach Anspruch 1 oder 2, dadurch gekennzeichnet, daß die Hornhautlinse mit den Stiften ein Spritzgußteil aus Kunststoff ist.

4. Vorrichtung nach einem der Ansprüche 1 bis 3, gekennzeichnet durch ein Leiterkabel (7), das um einen anderen Stift (2"), als der (2"), durch den die Kontaktleitung (6, 4) geführt ist, herum befestigt ist, und bei der Anwendung als Inaktive- oder Referenz-Elektrode dient.

FIG. 1

FIG. 2

FIG.3

FIG.4